Europäisches Patentamt

(19) European Patent Office

Office européen des brevets

(11) Veröffentlichungsnummer: **0 045 476**
**B1**

(12) # EUROPÄISCHE PATENTSCHRIFT

(45) Veröffentlichungstag der Patentschrift:
23.10.85

(21) Anmeldenummer: 81105956.7

(22) Anmeldetag: 29.07.81

(51) Int. Cl.⁴: **G 01 N 33/48**, G 01 N 33/52,
A 61 K 35/14, C 12 Q 1/00,
B 01 L 3/14, B 01 D 39/20

(54) Verfahren zur Abtrennung von Plasma oder Serum aus Vollblut.

(30) Priorität: 05.08.80 DE 3029579

(43) Veröffentlichungstag der Anmeldung:
10.02.82 Patentblatt 82/6

(45) Bekanntmachung des Hinweises auf die Patenterteilung:
23.10.85 Patentblatt 85/43

(84) Benannte Vertragsstaaten:
AT BE CH DE FR GB IT LI LU NL SE

(56) Entgegenhaltungen:
DE - A - 2 118 455
DE - A - 2 222 951
DE - A - 2 922 958
DE - A - 2 934 760
DE - B - 2 356 353
FR - A - 2 419 058
FR - A - 2 419 073
FR - A - 2 430 784
US - A - 3 791 933
US - A - 3 983 005

(73) Patentinhaber: BOEHRINGER MANNHEIM GMBH,
Patentabteilung, Abt. E Sandhofer
Strasse 112-132 Postfach 31 01 20,
D-6800 Mannheim 31 Waldhof (DE)

(72) Erfinder: Vogel, Peter, Dr.rer.nat., Schubertweg 5,
D-6944 Hemsbach (DE)
Erfinder: Braun, Hans-Peter, Dr.rer.nat., Rainweg 103,
D-5900 Heidelberg 1 (DE)
Erfinder: Berger, Dieter, Dr.rer.nat.,
Bensheimer-Strasse 45, D-6806 Viernheim (DE)
Erfinder: Werner, Wolfgang, Dr.rer.nat., Meissener
Weg 39, D-6800 Mannheim-Vogelstang (DE)

## Beschreibung

In der Klinischen Chemie ist die Abtrennung von Serum oder Plasma aus Blut von überragender Bedeutung, da praktisch nur aus diesen beiden die Analyse von gelösten Blutbestandteilen störungsfrei durchgeführt werden kann.

Die normale und gebräuchlichste Form der Abtrennung von Serum oder Plasma von den Erythrozyten ist die Zentrifugation. Diese ist jedoch insbesondere bei der Verwendung kleiner Probenmengen problematisch, auch ist die Trennung von Überstand und Blutkuchen nicht immer einfach, so daß hierfür eine ganze Reihe von Hilfsmitteln in der Literatur zu finden sind, z. B. DE-AS 2 559 242.

Von besonderer Problematik ist die Verwendung von Vollblut bei Schnelldiagnostica. Schnelldiagnostica sind reagentienhaltige saugfähige oder quellbare Träger, vorzugsweise aus Filterpapier, auf die eine geringe Menge, z. B. ein Tropfen der zu untersuchenden Flüssigkeit aufgebracht wird und bei denen aufgrund der überwiegend sehr kurzen Reaktion eine Farbänderung eintritt, die entweder visuell bewertet wird oder remissionsphotometrisch ausgemessen wird. Da trübe und gefärbte Lösungen wie Blut die Ablesung stören, hat es daher nicht an Versuchen gefehlt, Schnelldiagnostica für die direkte Verwendung von Vollblut zugänglich zu machen. Hierbei sind z. B. zu nennen das Überziehen von Testpapieren mit semipermeablen Membranen (US-PS 3 092 465) und die Verwendung von wasserquellbaren Filmen, in die nur die gelösten Bestandteile des Blutes, nicht aber die Erythrozyten eindringen können (DE-AS 1 598 153). Diese beiden Verfahren sind an sich brauchbar, allerdings nur bei Testen für niedermolekulare Bestandteile des Blutes, wie z. B. Glucose oder Harnstoff; höhermolekulare Bestandteile des Blutes, wie z. B. Lipide oder die an Serum-Protein gebundenen Substrate wie z. B. Bilirubin, können auf diese Weise nicht bestimmt werden, weil sie nicht in der Lage sind, in den Film einzudringen bzw. durch die semipermeable Membran hindurchzugelangen. Weiterhin sind Vorschläge bekannt geworden, diagnostische Mittel zum Abtrennen der Blutzellen mit Membranfiltern zu bedecken (DE-AS 2 222 951 und DE-OS 2 922 958). Der Nachteil dieser diagnostischen Mittel ist, daß durch Membranfilter das Blut nur sehr langsam und in geringer Menge durchdringen kann, weil sie leicht verstopfen und die Reaktion entsprechend lange dauert. Im Gegensatz zu den vorher genannten diagnostischen Mitteln, die bereits im Handel sind, sind »Schnellteste« der zuletzt geschilderten Art deshalb noch nicht im Handel erschienen.

Aus den DE-OS 2 908 721 und 2 908 722 ist weiterhin bekannt, daß man Lymphozyten, beziehungsweise Leukozyten, aus Blut abtrennen kann, wenn man das Blut über eine Schicht aus Kunstfasern mit mittleren Faserdurchmessern von 5−20 μm, beziehungsweise 3−10 μm, filtriert. Da die Erythrozyten aber überwiegend mit dem Plasma durch den Filter laufen, sind diese Filter nicht geeignet zur Gewinnung von Plasma. Rein spekulativ werden darüber hinaus Kohlenstoffasern, Glasfasern und Metallfasern genannt.

Aus der DE-AS 2 356 353 ist weiter bekannt, daß man koagulierte Aggregate aus Blutkonserven, die zur Blutübertragung dienen sollen, über einen Filter entfernen kann, und die nicht koagulierte Partikel, d h. Leukozyten und Erythrozyten, durchgelassen werden, wenn als Filter ein Faserfilter aus Polyester oder Polyamid mit einer Dichte von etwa 0,7 g/cm$^3$ verwendet wird, welches durch Komprimieren und Erhitzen auf den Erweichungspunkt eines Teils der Fasern verfestigt ist. Plasma kann mit einer solchen Vorrichtung nicht vorgenommen werden.

In der DE-AS 2 118 455 werden ferner Teststreifen beschrieben, bei denen die reagenzhaltigen Indikatorschichten mit einem feinen Netz eine Maschenweite von ca. 20−100 μm hat, ist es zur Abtrennung von feineren Partikeln nicht geeignet, so daß mit solchen Teststreifen nur klare Lösungen (Harn, Serum) untersucht werden können.

Aufgabe der Erfindung war es daher, ein einfaches Verfahren zum Abtrennen von Plasma oder Serum aus Vollblut zu finden, das ohne Zentrifugieren rasch und sicher kleine Mengen Blut trennt und insbesondere als Probenvorbereitung für diagnostische Zwecke geeignet ist.

Es wurde nun gefunden, daß die Abtrennung von Plasma bzw. Serum aus Vollblut schnell und einfach und in genügender Menge erfolgt, wenn man das Blut langsam durch eine Schicht aus Glasfasern mit einem mittleren Durchmesser von 0,2−2,5 μm und einer Dichte von 0,1−0,5 g/cm$^3$ sickern läßt und das abgetrennte Plasma oder Serum gewinnt, wobei das Volumen des abzutrennenden Plasmas oder Serum höchstens 50%, vorzugsweise weniger als 30%, vom Saugvolumen der Glasfaserschicht beträgt.

Diese Tatsache muß um so mehr als überraschend angesehen werden, als in der oben erwähnten DE-AS 2 222 951 die Verwendung von Glasfasermatten zur Abtrennung von weißen Blutzellen schon beschrieben ist, jedoch für die Abtrennung der Erythrozyten die zusätzliche Verwendung von Membranfiltern als unbedingt notwendig gefordert wird.

Weiterbildungen der Erfindung sind in den abhängigen Ansprüchen näher gekennzeichnet.

Die Glasfasern können lose gestapelt sowie in Form von Papieren, Vliesen, oder Filzen, aber auch gehalten durch eine äußere Form, in jeder gewünschten Gestalt Verwendung finden.

Die so gestalteten Glasfasern können als Abdeckung für eines der oben beschriebenen Schnelldiagnostica dazu dienen, daß dieses diagnostische Mittel, für dessen Verwendung bisher die vorherige Gewinnung von Serum oder Plasma notwendig war, jetzt für die direkte Verwendung von Vollblut geeignet ist.

Weiterhin können mit Glasfasern gefüllte Säulen, Filternutschen oder andere geeignete Gefäße auch dazu verwendet werden, durch einfaches Durchlaufen von Blut Serum oder Plasma ohne Zentrifugation zu gewinnen und diese in geeigneter Weise für diagnostische Mittel bereitzustellen, da das Serum bzw. Plasma schneller durch eine solche Schicht durchläuft, als die Erythrozyten und Leucozyten.

Die für das erfindungsgemäße Verfahren verwendbaren Glasfasern können aus Fasern unterschiedlichen Durchmessers bestehen. Das Glasmaterial kann aus alkalihaltigem oder alkalifreiem Borosilikatglas, aber auch aus reinem Quarzglas bestehen. Fasermaterial aus anderen technischen Gläsern, z. B. borfreien Alkaligläsern, Kristallglas, Bleiglas u. a. befinden sich in den notwendigen Abmessungen der Fasern nicht im Handel und konnten daher nicht untersucht werden. Es wird aber davon ausgegangen, daß auch sie geeignet sind. Der mittlere Durchmesser der Glasfasern kann zwischen 0,5 μm und 2,5 μm, insbesondere zwischen 0,5 μm und 1,5 μm, liegen. Die Faserdurchmesser können entsprechend der Herstellung stark streuen, sollten jedoch eine Obergrenze von ca. 10 μm nur ausnahmeweise überschreiten. Ihre Länge ist nur durch die Art der Stapelung begrenzt, hat aber ansonsten keinen Einfluß. Je nach Art der Stapelung werden Dichten von 0,1 – 0,5, üblicherweise 0,2 – 0,4 g/cm³ gefunden.

Weiterhin können die Glasfasern untereinander, aber auch mit Fasern aus anderen Materialien, vermischt werden, wodurch der innere Zusammenhalt der Fasern verbessert werden kann. Geeignet sind z. B. synthetische Fasern wie Polyester, Polyamid u. a., aber auch Fasern aus Polyethylen, Polypropylen und anderen nachträglich durch Wärme verformbaren Kunststoffen. Die Zusätze können auch höhere (10 – 20 μm) Durchmesser aufweisen, so lange ihre Menge nicht so groß ist, daß sie die Abtrennung durch die erfindungsgemäßen, feineren Glasfasern beeinflussen.

Weiterhin können die Glasfasern durch Zusatz von anorganischen Bindemitteln (z. B. Wasserglas) oder organischen Bindemittel (z. B. Polyvinylacetat, Polyvinylpropionat, Polyacrylsäureester u. a.) verfestigt werden, durch die die Glasfasern an den Berührungsstellen verklebt werden.

Besonders bevorzugt ist es, bei den erfindungsgemäßen Verfahren das erhaltene Plasma direkt in diagnostische Mittel einzubringen, bzw. die Glasfaserschicht in das diagnostische Mittel zu inkorporieren.

Die Glasfasern können in diesen diagnostischen Mitteln auch Reagenzien enthalten, die die Hämolyse der Erythrozyten verhindern, ferner Reagenzien, die die Gerinnung hemmen oder fördern sowie Reagenzien, die in der Indikatorschicht benötigt werden, mit den dortigen Reagenzien aber unverträglich sind. Diese letzteren Reagenzien können natürlich auch in allen Schichten untergebracht werden, die zwischen Glasfaser und Indikatorschicht liegen.

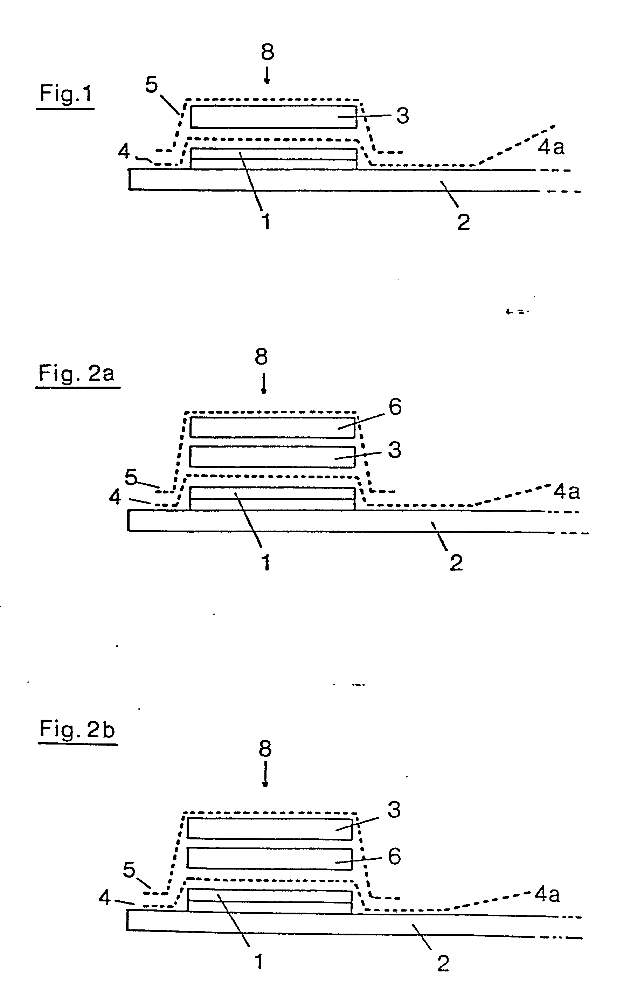

Den Aufbau eines solchen diagnostischen Mittels erläutert Fig. 1. Auf einer steifen Unterlage 2 ist die Reaktionsschicht 1 des Schnelldiagnosticums aufgeklebt. Dicht über der Reaktionsschicht ist eine dünne, für Flüssigkeiten durchlässige formstabile Trennschicht 4 aufgebracht, die aus einem Netzwerk aus gewebten oder verfilzten Kunststoff-Fäden besteht und die auf beiden Seiten der Reaktionsschicht in leicht lösbarer Weise auf die Grundfolie in der Art geklebt ist, daß ein leicht zu fassender Griff 4a am längeren Teil der Unterlage freibleibt. Oberhalb der Reaktionsschicht ist nun das Glasfaserpapier 3 aufgebracht. Dieses wird durch ein weiteres Netzwerk 5 in seiner Lage fixiert, das wie die Trennschicht 4 oberhalb und unterhalb der Reaktionsschicht angeklebt ist.

Die Reaktionsschicht 1 kann aus einem imprägnierten saugfähigen Träger oder quellbarem oder porösem Kunststoffilm bestehen. Als Unterlage 2 dient vorzugsweise eine dickere Kunststoffolie, ein fester Karton, eine Glasplatte oder ein anderes stabiles Trägermaterial.

Nach Aufbringen eines Bluttropfens 8 auf die obere Seite des Schnelldiagnosticums wird im Glasfaserpapier das Plasma von den Erythrozyten und Leucozyten abgetrennt. Das auf diese Weise abgetrennte Plasma gelangt über die Trennschicht 4 in die Reaktionszone 1 des diagnostischen Mittels. Nach einer angemessenen Zeit, in der das Plasma in die Reaktionszone eingedrungen ist, wird die Trennschicht an ihrem freien Ende ergriffen und mit dem Glasfaserpapier und dem Netzwerk 5 abgetrennt. Anschließend kann die Reaktionsschicht, in der nun die Nachweisreaktion stattfindet, visuell oder remissionsphotometrisch ausgewertet werden.

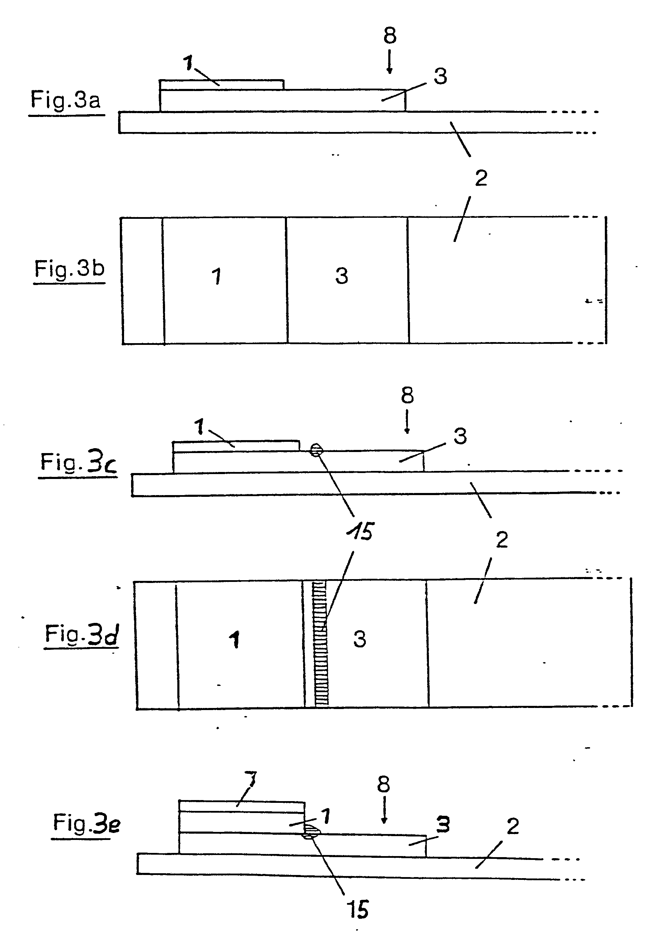

Einen weiteren möglichen Aufbau eines Schnelldiagnosticums verdeutlicht Fig. 2, wobei zusätzlich zu dem oben beschriebenen Aufbau eine oder mehrere für Flüssigkeiten aller Art durchlässige Schichten 6 in der Art aufgebracht sind, daß sie entweder oberhalb (Fig. 2a) oder unterhalb (Fig. 2b) der Glasfaserpapiere zu liegen kommen. Diese zusätzlichen Schichten können mit Reagenzien imprägniert sein, die entweder leicht löslich sind und zusammen mit dem Plasma in die Reaktionszone gelangen oder aber weniger löslich sind und eine oder mehrere Vorstufen der Nachweisreaktion bereits außerhalb der endgültigen Reaktionszone 1 ablaufen lassen.

In Fig. 3 wird ein anderer Aufbau (Fig. 3a Seitenansicht, Fig. 3b Aufsicht) des Schnelldiagnosticums beschrieben, bei dem das Glasfaserpapier 3 direkt auf die Unterlage 2 aufgeklebt ist. Auf einen Teil des Glasfaserpapieres ist die Reaktionsschicht 1 aufgebracht. Auf den freibleibenden Teil des Glasfaserpapiers wird das Blut 8 aufgetragen. Das sich im Glasfaserpapier von den Erythrozyten abtrennende Plasma diffundiert im Glasfaserpapier zu der Reaktionsschicht und in diese hinein. Die bei der Reaktion entstehenden Reaktionsfarben können von der Oberseite des Schnelldiagnosticums beobachtet und ausgewertet werden. Die Reaktionsschicht kann direkt durch Bedrucken oder Beschichten

3

auf das Glasfaserpapier aufgebracht werden. Sie kann aber auch in Form eines ganz- oder teilimprägnierten saugfähigen Trägers auf das Glasfaserpapier aufgeklebt werden.

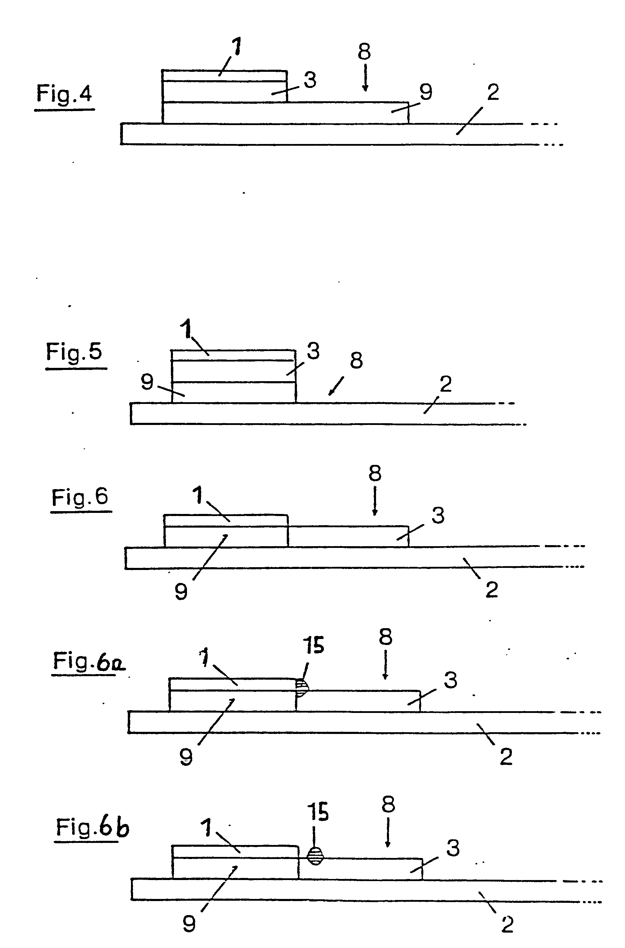

Weiterhin kann, wie in Fig. 4 und Fig. 5 beschrieben, das diagnostische Mittel so aufgebaut sein, daß auf der Unterlage 2 zuerst ein saugfähiges Material 9 wie z. B. Cellulsoepapier oder ein Kunststoffvlies und oberhalb dieses Materials das Glasfaserpapier 3 und die Reaktionsschicht 1 aufgeklebt sind. Dabei kann das saugfähige Material 9 die gleiche Fläche wie die Reaktionsschicht einnehmen (Fig. 5) oder aber eine größere Fläche, so daß das Material 9 eine Fläche freiläßt (Fig. 4). Das Blut wird auf die freibleibende Fläche des saugfähigen Materials (Fig. 4) oder direkt neben das saugfähige Material (Fig. 5) aufgetropft und von diesem schnell aufgenommen und unter das Glasfaserpapier gesaugt. Anschließend wird durch die Saugfähigkeit des Glasfaserpapieres Blut durch das Glasfaserpapier nach oben gesaugt, wobei die Abtrennung der Erythrozyten erfolgt und Plasma in die Reaktionschicht 1, gelangt. Die Reaktion wird wie in Fig. 3 von der Oberseite des Schnelldiagnosticums beobachtet.

Fig. 6 zeigt einen weiteren Aufbau eines für die direkte Verwendung von Vollblut geeigneten Schnelldiagnosticums. Dieses ist so aufgebaut, daß auf der steifen Unterlage 2 nebeneinander eine saugfähige Schicht 9, die z. B. aus einem Cellulosepapier oder aus einem cellulosehaltigen Kunstfaservlies besteht und eine Glasfaserschicht 3 aufgebracht sind. Die beiden Schichten sollen einen engen Kontakt aufweisen. Auf der Oberfläche der saugfähigen Schicht 9 befinden sich die für das Schnelldiagnosticum notwendigen Nachweisreagenzien, die z. B. durch Beschichten mit einem offenen Film gemäß DE-OS 2 910 134 aufgebracht sein können. Beim Auftragen des Bluttropfens auf die von der Reaktionszone entferntere Seite der Glasfaserschicht findet die Trennung Plasma-Erythrozyten so statt, daß zuerst das Plasma mit seiner Front an der Trennstelle zu der saugfähigen Schicht 9 anlangt und sofort von dieser aufgesaugt wird. Durch Kapillarkräfte gelangt dann das Plasma in die Reaktionsschicht 1, wo sich die Nachweisreaktion z. B. durch eine von oben sichtbare Farbveränderung bemerkbar macht.

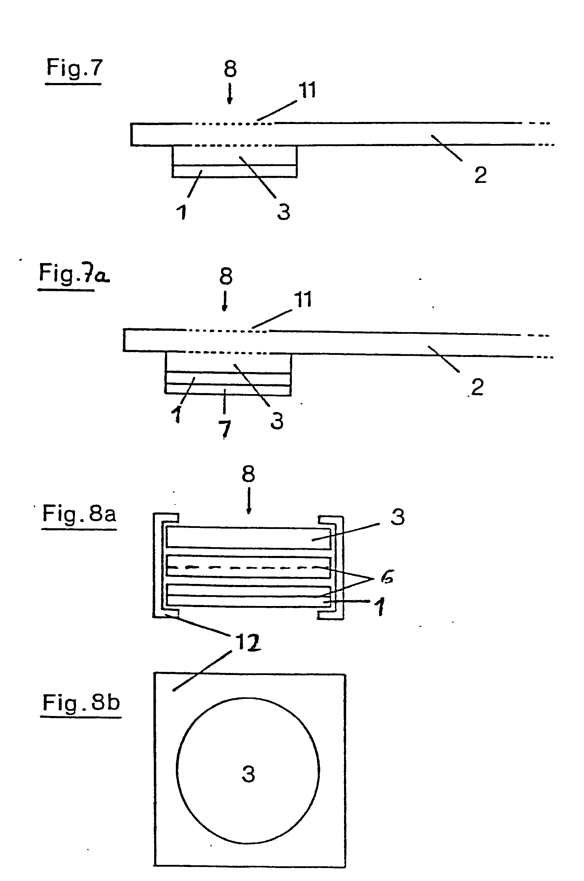

Weiterhin kann das erfindungsgemäße diagnostische Mittel in der in Fig. 7 beschriebenen Form hergestellt werden. Dabei wird das Glasfaserpapier 3 auf eine Unterlage 2 aufgeklebt. Die Unterlage hat an der Berührungsstelle ein oder mehrere Löcher 11. Auf der anderen Seite ist eine Reaktionsschicht 1 direkt oder durch Kleben auf das Glasfaserpapier aufgebracht. Das Blut 8 wird so auf das diagnostische Mittel aufgebracht, daß es durch das Loch (oder die Löcher) auf das Glasfaserpapier 3 gelangen kann. Das im Glasfaserpapier abgetrennte Plasma trifft nun auf die Reaktionsschicht 1 und führt zu der Reaktion, die visuell oder remissionsphotometrisch an deren Oberfläche ausgewertet werden kann. Die Reaktionsschicht ist dabei durch eine durchsichtige Abdeckschicht 7 geschützt.

Die Reaktionsschicht 1 kann sowohl aus einer, z. B. auf die Glasfasermatte aufgedruckten Schicht bestehen, sie kann aber auch aus einem mehrschichtigen Element bestehen, bei dem die Schichten verschiedene Reagenzien enthalten können und/oder mehrere Funktionen erfüllen können, wie z. B. in der DE-OS 2 922 958 beschrieben.

Fig. 8a zeigt in der Seitenansicht und Fig. 8b zeigt in der Aufsicht einen weiteren Aufbau eines solchen diagnostischen Mittels, bei dem die aus Glasfasern bestehende Schicht 3 sowie eine oder mehrere für die Reaktion notwendigen Schichten 6 durch eine vorgefertigte Form 12 zusammengehalten werden. Das Blut wird hierbei auf die Seite des Glasfaserfilters aufgetropft. Das abgetrennte Plasma gelangt anschließend in die Reaktionsschicht 1 wobei die Indikatorreaktion auf der dem Glasfaserfilter gegenüberliegenden Seite visuell oder remissionsphotometrisch ausgewertet wird.

Aus zeichentechnischen Gründen sind in einigen Figuren die verschiedenen Schichten teilweise mit einem Zwischenraum gezeichnet. In der praktischen Ausführung liegen die Schichten aufeinander, so daß Flüssigkeiten ungehindert übertreten können. Weiterhin sind die Reaktionsschichten 1 und 6 quer unterteilt, um anzudeuten, daß sie auch aus mehreren übereinanderliegenden Schichten bestehen können.

Die Blutabtrennung mit Mitteln gemäß Fig. 3a, 3b, 4 und 6 läßt sich wesentlich verbessern, wenn man auf die Glasfaser 3 neben oder in der Reaktionsschicht 1 eine hydrophobe Barriere 15 aufbringt, die teilweise in das Volumen der Glasfaser 3 hineinragt. Die Fig. 3c, 3d, 6a und 6b zeigen diese Barriere 15 und entsprechen im übrigen dem Aufbau gemäß Fig. 3, 4 und 6. Diese Barriere 15 verhindert nicht nur, daß sich Blut 8 an der Oberfläche der Glasfaser zuerst ausbreitet und den Indikatorbezirk verunreinigt, sie verbessert auch entscheidend die Trennwirkung der Glasfaser. Es kann deshalb mit relativ kleinen Glasfaserpapieren 3 gearbeitet werden, was zur Folge hat, daß wesentlich kleinere Blutmengen benötigt werden. Die Barriere kann auf herkömmliche Weise, z. B. aus Düsen oder mit Rädern, aufgebracht werden. Das hydrophobe Material für die Barriere kann z. B. ein Schmelzkleber oder ein herkömmlicher Lösungsmittelkleber oder Wachs sein.

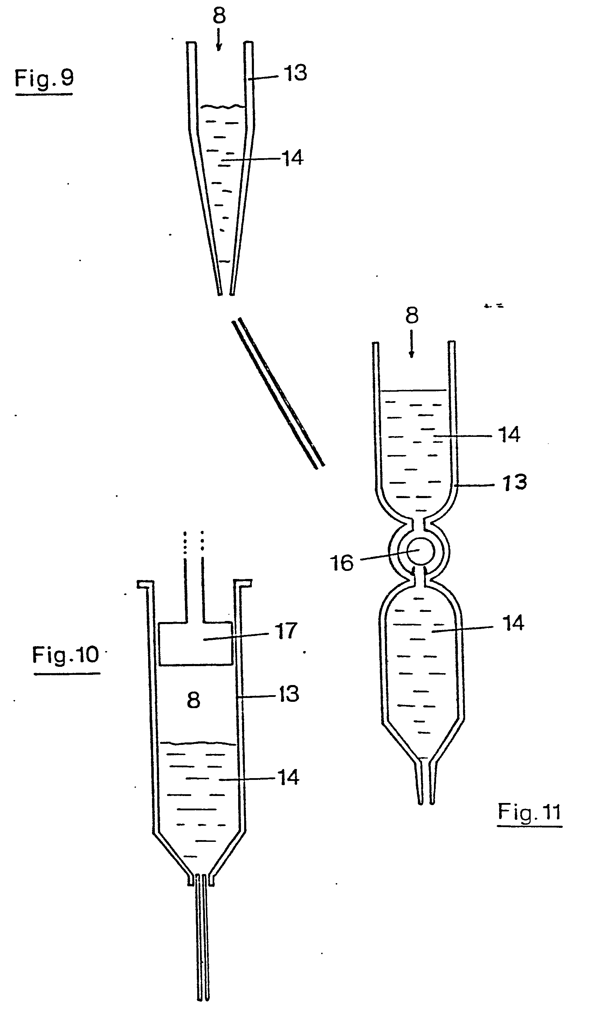

Des weiteren beschreibt Fig. 9 den Aufbau einer erfindungsgemäß verwendbaren Vorrichtung, die Plasma von den Erythrozyten des Vollblutes ohne Zentrifugieren abtrennt und für diagnostische Zwecke bereitstellt. Dazu wird ein Rohr oder ein Gefäß 13, das z. B. die beschriebene Form hat, mit den oben beschriebenen Glasfasern 14 gefüllt. Das Blut 8 wird oben in das Gefäß eingefüllt. Auf dem Weg nach unten wird nun durch die Glasfasern das Plasma von den Erythrozyten des Blutes abgetrennt. Das sich im unteren Teil des Gefäßes 13 sammelnde Plasma kann nun z. B. durch eine »end-to-end«-Kapillare aufgenommen oder abgesaugt werden und direkt einem anderen diagnostischen Verfahren zugeführt werden.

Einen anderen erfindungsgemäß verwendbaren Aufbau beschreibt die Fig. 10, wobei das zur Abtrennung der Erythrozyten geeignete Gefäß 13 die Form einer Kolbenspritze aufweisen kann, die im unteren Teil mit Glasfasern 14 dicht gefüllt ist. Das Blut 8 wird in das oben offene Gefäß eingebracht. Nach erfolgter Trennung von Erythrozyten und Plasma, wobei das Plasma sich im unteren Gefäßteil sammelt, kann durch Einführen und vorsichtiges Drücken des Kolbens 17 zuerst das Plasma aus dem Spritzkörper herausgedrückt werden.

Das erfindungsgemäße Verfahren zur Plasma-Gewinnung kann, wie in Fig. 11 beschrieben, auch mit einem Gefäß 13 durchgeführt werden, das durch ein in einer Richtung durchlässiges Ventil 16 in zwei Teile getrennt ist und mit den oben beschriebenen Glasfasern gefüllt ist. Das Blut 8 wird oben in das Gefäß eingefüllt. Das Plasma sammelt sich nach der Abtrennung von den Erythrozyten im unteren Teil des Gefäßes und kann nun durch Zusammenpressen des unteren Gefäßteiles entleert werden. Dabei verhindert das Ventil 16, daß das Plasma in den oberen, die Blutzellen enthaltenden Teil zurückströmt.

Weiterhin kann das erfindungsgemäße Verfahren mit einer Anordnung durchgeführt werden, die durch Fig. 1 beschrieben wird, wobei die auf die Unterlage 2 geklebte Reaktionsschicht 1 aus einem definiert saugenden Material besteht, so daß beim Auftragen von Blut ein definiertes Plasmavolumen in die Schicht 1 gelangt. Nach dem Abtrennen der Schichten 3, 4 und 5 kann das Plasma, d. h. die zu analysierende Substanz durch ein Lösungsmittel eluiert werden. Die Elution des Plasmas und die Analyse kann sofort, aber auch — je nach zu analysierender Substanz — zu einem späteren Zeitpunkt an anderem Ort durchgeführt werden. Falls die Analyse erst später durchgeführt werden soll, kann es vorteilhaft sein, das Plasma zunächst, beispielsweise mit warmer Luft oder durch Gefriertrocknen, einzutrocknen. Es ist weiterhin möglich, getrennt von dem Bezirk für das Aufbringen der Probe, einen oder mehrere Bereiche vorzusehen, die Reagenzien enthalten, so daß beim Eluieren mit einem Lösungsmittel das ganze Reaktionsgemisch gleichzeitig eluiert wird.

Wenn die Reaktionsfarben nicht nur visuell ausgewertet werden sollen, sondern in Remissionsphotometern gemessen werden sollen, ist es zweckmäßig, die Reaktionsschicht 1 mit einer Abdeckschicht 7 zu bedecken, um eine Verschmutzung der Meßanordnung zu vermeiden. Ist die Reaktionsschicht 1 ein Film etwa gemäß DE-OS 2 910 134 oder DE-PS 1 598 153, so ist es zweckmäßig diese direkt auf die Abdeckschicht 7 zu beschichten und dann beide gemeinsam zu montieren. Eine mögliche Ausführungsform zeigt Fig. 3e. Naturgemäß ist die Abdeckschicht 7 auch anwendbar auf andere Ausführungsformen, wie es in Fig. 7a gezeigt ist, die im übrigen dem Aufbau gemäß Fig. 7 entspricht.

Es hatte sich weiterhin als vorteilhaft erwiesen, Anordnungen zu wählen, bei dem die Reaktionsschicht 1 zunächst nicht mit der Glasfaser 3 bzw. der saugfähigen Schicht 9 in flüssigkeitsleitender Berührung ist, sondern daß diese Berührung erst dann herbeigeführt wird, wenn sich die genannten Schichten vollständig mit Plasma bzw. Serum gefüllt haben. Die Vorteile dieser Anordnungen sind, daß das Plasma bzw. Serum mit der Reaktionsschicht 1 zu einem vorher bestimmbaren exakten Zeitpunkt in Berührung gebracht werden können. Ferner erfolgt diese Berührung über die ganze Fläche, so daß Chromatographieeffekte, wie sie bei den vorstehenden Anordnungen gegebenenfalls auftreten können, ausgeschlossen sind. Die Tatsache, daß zwischen Aufgeben des Blutes 8 und Beginn der Reaktion in der Reaktionsschicht 1 eine vorher bestimmbare Zeit gelegt werden kann, ist von großer Bedeutung bei Reaktionen die unter besonders kontrollierten Bedingungen ablaufen müssen. So kann bei der Bestimmung von Enzymen, die bei besonders konstanter Temperatur ablaufen muß, die Reaktion erst gestartet werden, wenn das diagnostische Mittel hinreichend konstant temperiert ist. Ebenso können in den Zonen von 3 und 9, in denen sich das Plasma sammelt, Reagenzien untergebracht werden, die den nachzuweisenden Stoff in zeitabhängiger Reaktion in einen bestimmten Zustand bringen, d. h. eine Vorreaktion ablaufen lassen. Ein Beispiel hierfür ist die Aktivierung der Creatin-Kinase mit N-Acetylcystein.

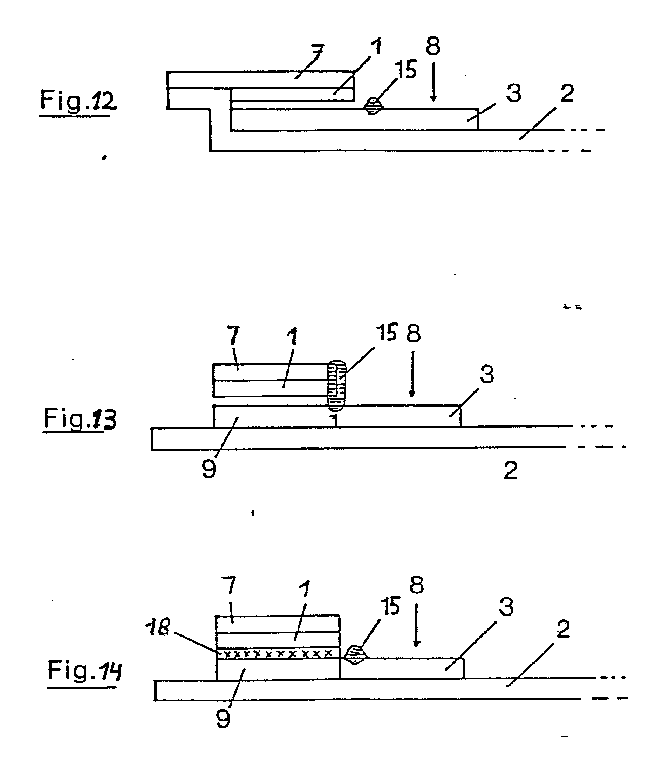

Fig. 12, 13 und 14 beschreiben verschiedene mögliche Anordnungen, wobei in Fig. 13 die hydrophobe Barriere 15 gleichzeitig als Befestigung der Schichten 1 und 7 dient. Die Bezeichnungen und Zusammensetzungen der übrigen Schichten entsprechen den Fig. 3 bis 6.

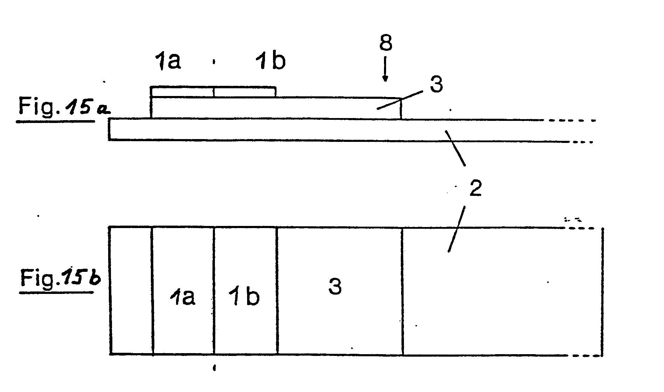

In Fig. 14 ist die Verwendung eines hydrophoben Netzes 18 zwischen Reaktionsschicht 1 und Glasfaser 3 bzw. Saugschicht 9 beschrieben. Dieses hydrophobe Netz schützt die Anordnung vor unbeabsichtigtem leichtem Berühren und läßt den Flüssigkeitskontakt erst bei stärkerem Anpressen eintreten. Dies trägt zu einer verbesserten Praktikabilität bei.

Natrugemäß ist es möglich, daß die Reaktionsschicht 1 aus 2 oder mehreren voneinander verschiedenen Bezirken bestehen kann. Diese können entweder die gleiche Substanz in verschiedenen Konzentrationsbereichen oder aber verschiedene Substanzen nachweisen, wenn die Rezepturen entsprechend ausgewählt sind. Ferner sind auch Anordnungen denkbar, bei denen gleichzeitig verschiedene Reaktionsschichten von dem Plasma, das aus einer Auftropfstelle herrührt, benetzt werden. Es sind hier die verschiedensten Formen denkbar, längliche, kreisförmige und dergleichen.

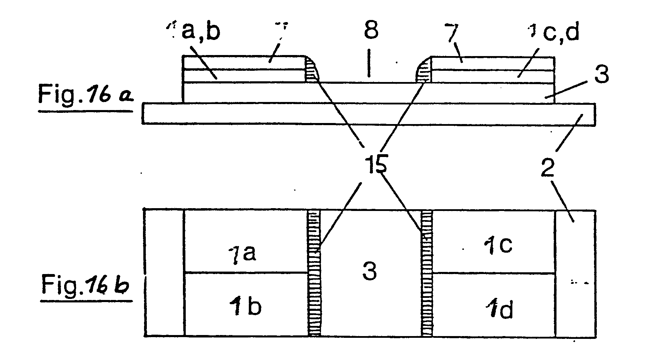

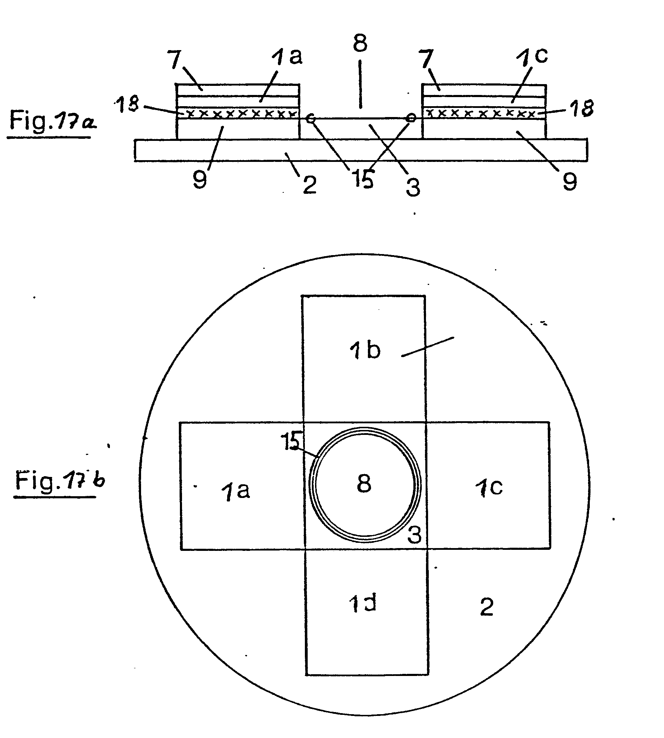

In Fig. 15a bis 17b sind einige mögliche Anordnungen dargestellt, wobei die verschiedenen Testbezirke mit 1a bis 1d unterschieden wurden und die übrigen Bezeichnungen den vorstehenden Fig. entsprechen.

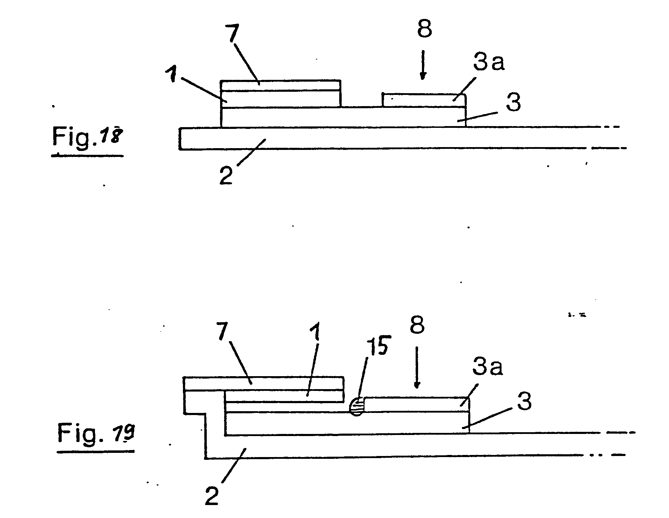

Die Blutabtrennung kann gleichfalls verbessert werden, wenn auf der Glasfaser 3 an der Blutauftropfstelle noch ein weiterer Glasfaserbezirk 3a aufgebracht wird. Hierbei kann 3a und 3 aus dem gleichen Material bestehen, es kann aber auch für 3a ein Material anderer Dicke, bzw. mit anderem Faserdurchmesser gewählt werden. Fig. 18 und 19 zeigen mögliche Anordnungen, die im übrigen den Fig. 3 und 12 entsprechen.

In den folgenden Beispielen soll die Erfindung näher erläutert werden:

Beispiel 1

Cholesterin-Teststreifen

| | |
|---|---|
| 0,117 g | Methylhydroxypropylcellulose (Culminal MHPC 8000) |
| 7,000 g | Titandioxid |
| 0,138 g | $KH_2PO_4$ |
| 0,479 g | $Na_2HPO_4 \cdot H_2O$ |
| 3400 U | Cholesterinesterase |
| 5000 U | Cholesterinoxidase |
| $7 \cdot 10^4$ U | Peroixdase |
| 0,476 g | Natriumdioctylsulfosuccinat |

werden in 70 ml Wasser gelöst. Dann werden nacheinander

| | |
|---|---|
| 14,0 g | Cellulose |
| 8,4 g | Polyvinylpropionat-Dispersion |

homogen eingearbeitet. Zuletzt wird

| | |
|---|---|
| 0,66 g | 3,3',5,5'-Tetramethylbenzidin gelöst in |
| 1,6 ml | Aceton |

zugegeben. Dieser Ansatz wird etwa 300 µm dick auf eine glatte Kunststoffolie beschichtet und nach dem Trocknen bei 60°C–70°C in 6 mm breite Streifen geschnitten. Diese Streifen werden anschließend zusammen mit einem 60 µm starken Netzwerk aus Nylon und einem ebenfalls in 6 mm breite Streifen geschnittenen Glasfaserpapier (Glasfaserfilter Nr. 3362 von Schleicher & Schüll, Papierdicke 0,47 mm, Dichte 0,27 g/cm³, mittlerer Faserdurchmesser ca. 2,5 µm auf eine Polyesterfolie geklebt. Anschließend wird sie in 6 mm breite Streifen zerschnitten.

Betüpfelt man die Oberseite des Teststreifens mit 40 µl Blut und entfernt nach 1 Minute das Glasfaserpapier mit dem restlichen Blut zusammen mit dem Netzwerk durch Abreißen, dann bildet sich innerhalb von 3 Minuten auf dem Testbezirk eine Reaktionsfarbe, die derjenigen entspricht, die man erhält, wenn anstelle des Blutes mit dem abzentrifugierten Plasma des gleichen Blutes getüpfelt wird.

Beispiel 2

Cholesterin-Test

| | |
|---|---|
| 0,45 g | $KH_2PO_4$ |
| 1,55 g | $Na_2HPO_4 \cdot H_2O$ |
| $1,5 \cdot 10^4$ U | Cholesterinesterase |
| $1 \cdot 10^4$ U | Cholesterinoxidase |
| $3 \cdot 10^5$ U | Peroxidase |
| 2,0 g | Na-Dioctylsulfosucciant |
| 6,9 g | Natrium-Alginat (Algipon) |

werden in 250 ml Wasser gelöst, dann werden noch

| | |
|---|---|
| 2,0 g | 3,3',5,5'-Tetramethylbenzidin |

gelöst in 15 ml Aceton dazugegeben. Anschließend werden noch

| | |
|---|---|
| 20,0 g | Kieselgur |

homogen verteilt. Diese Reaktionsmasse wird in 6 mm breiten Streifen mit einer Siebdruckmaschine (Gewebe: 190 µm) auf ein Glasfaserpapier in der im Beispiel 1 beschriebenen Form aufgebracht. Das bedruckte Glasfaserpapier wird bei 60°C–80°C getrocknet und so in 12 mm breite Streifen geschnitten, daß die bedruckte Reaktionszone die eine Hälfte des Streifens ausmacht. Dieser Streifen auf das Ende einer Polyesterfolie geklebt und diese quer zu den Glasfaserpapieren in 6 mm breite Streifen geschnitten. Wenn nun auf die der Reagenzschicht abgewendeteten Kante des unbeschichteten Glasfaserpapieres 40 µl Blut aufgetropft werden, diffundiert das Plasma unter die Reaktionszone. Diese nimmt in Abhängigkeit von der Cholesterinkonzentration des Blutes eine unterschiedlich stark gefärbte blaue Reaktionsfarbe an. Die Intensität der Reaktionsfarbe entspricht derjenigen, die man erhält, wenn man anstele des Blutes mit dem aus dem gleichen Blut gewonnenen Serum oder Plasma tüpftelt.

In gleicher Weise können auch die in der folgenden Tabelle 1 aufgeführten Papiere verwendet werden.

Tabelle 1

Glasfaserfilterpapiere zur Plasmaabtrennung

| Hersteller | Typ | Papier Faserdurchmesser, (µm) | mittl. Faserdurchmesser, (µm) | Flächengewicht, (g/m²) | Dicke, (µm) | Dichte (g/cm³) |
|---|---|---|---|---|---|---|
| Nuclepore | P 300 | 1 − 3 | 1,5 | 25 | 89 | 0,275 |
| Schleicher & Schüll | 3362 | 1 − 7 | 2,5 | 127 | 472 | 0,269 |

## Beispiel 3

### Cholesterintest

Eine Reagenzienmasse, bestehend aus

| 16,0 g | Cellulsoe M + N Ac 10 |
| 86,0 g | einer 0,2%igen Lösung von Methylhydroxypropylcellulose (Culminal MHPC 8000) |
| 0,32 g | Netzmittel (Marlon) |
| 0,68 g | Netzmittel (Na-Dioctylsulfosuccinat) |
| 12,0 g | Polyvinylpropionat-Dispersion (Propiofan 70 D) |
| 0,48 g | Tetramethylbenzidin |
| 10,0 g | Titandioxid |
| 9600 U | Cholesterinesterase |
| 7200 U | Cholesterinoxidase |
| 1,04 · 10⁴ U | Peroxidase |
| 0,01 g | Gallussäure |

wird in einer Dicke von 0,2 mm auf ein hydrophobes Polyestervlies (Reemay 2033 von Du Pont) beschichtet und bei 60° C getrocknet. Anschließend werden ein 6 mm breiter Streifen dieser Beschichtung und ein 12 mm breiter Streifen eines Glasfaserfilters (z. B. das Filter 3362 von Schleicher & Schüll) so nebeneinander auf einen festen Plastikstreifen geklebt, daß das Glasfaserfilter sehr eng an das beschichtete Vlies anstößt. Wenn von diesem Plastikstreifen quer 6 mm breite Streifen abgeschnitten werden, dann erhält man Teststreifen, bei denen nach Auftropfen von ca. 50 µl Vollblut auf die vom Reagenzienvlies entfernte Seite des Glasfaserfilters nach kurzer Zeit nur reines Plasma in das Reagenzienvlies übertritt und zu einer blauen Reaktionsfarbe führt, deren Intensität mit der Konzentration des Cholesterins im Blut zunimmt.

## Beispiel 4

### Separate Plasmagewinnung

Ein sich nach unten konisch verjüngendes Kunststoffgefäß (die Kunststoffspitze einer Kolbenhubpipette, Länge 5 cm, Dicke 0,5 cm) wird zu ²/₃ mit Glasfasern gemäß der folgenden Tabelle 2 locker gefüllt, wobei Schüttdichten von 0,1 − 0,4 g/cm³ erhalten werden. Nachdem der obere freie Teil mit Blut gefüllt wurde, diffundiert das Serum in die Gefäßspitze. Von dort kann eine »end-to-end«-Kapillare mit 15 µl Inhalt durch Heranführen an die Pipettenspitzenöffnung gefüllt werden. Das auf diese Weise gewonnene Plasma kann nun direkt jedem beliebigen analytischen Verfahren zugeführt werden.

Tabelle 2

Untersuchung des Trennvermögens verschiedener Glasfasern in einem Versuchsaufbau nach Beispiel 4

Glasfasern: Eigenschaften, Trennvermögen Erythrozyten/Plasma

| Johns-Manville USA | VEB Trisola DDR | Durchmesser Bereich | (µm) Mittel-wert | Faser-länge µm | Ober-fläche m²/g | Trennung Erythrozyten/Plasma |
|---|---|---|---|---|---|---|
| 100 | | 0,2 −0,29 | 0,25 | 300 | 5,1 | + |
| 102 | | 0,3 −0,33 | 0,32 | | 4,5 | + |
| 104 | | 0,34−0,48 | 0,4 | 800 | 3,12 | + |
| 106 | | 0,49−0,58 | 0,54 | 1000 | 2,6 | + + + |
| | U 60 | 0,51−0,64 | 0,58 | | − | + + |
| 108 A | | 0,59−0,88 | 0,74 | 1200 | 1,72 | + + |
| | U 70 | 0,65−0,76 | 0,71 | | | + + |
| | U 80 | 0,77−0,93 | 0,85 | | | + + + |
| | U 90 | 0,94−1,19 | 1,07 | | | + + |
| | U 100 | 1,2 −1,44 | 1,32 | | | + + + |
| 108 B | | 0,89−2,16 | 1,53 | | 0,71 | + + + |
| | U 156 | 1,45−2,49 | 1,97 | | − | + |
| 110 | | 2,17−3,10 | 2,6 | | 0,49 | − |
| 112 | | 2,6 −3,8 | 3,2 | 1900 | 0,40 | − |
| | F | 2,5 −4,0 | 3,3 | | | − |

+ befriedigend    + + gut    + + + sehr gut    − negativ

⊗ 80% der Fasern liegen in diesem Bereich.

## Beispiel 5

### Effekte einer hydrophoben Barriere

Zur Verdeutlichung der Effekte einer hydrophoben Barriere 15, wurden Vorrichtungen gemäß Fig. 14 hergestellt, die aus einer durchsichtigen Polycarbonat-Trägerfolie 2 von 6 mm Breite und 0,3 mm Dicke, einer 9 × 6 mm großen saugfähigen Schicht 9 aus 0,09 mm dickem Glasfaserpapier, einem hydrophoben Nylonnetz 18 von 0,075 mm Dicke und einer transparenten Abdeckfolie 7 abgedeckt sind. In saugfähigem Kontakt mit der Schicht 9 ist ein Glasfaserpapier 3 auf dem Träger befestigt, das aus Glasfaserpapier (Schleicher & Schüll, Nr. 3362, Dicke 0,47 mm) von 6 mm Breite und der in der folgenden Tabelle angegebenen Länge besteht. Jeweils eine Hälfte dieser Vorrichtungen wird an der Berührungsstelle zwischen den Schichten 3, 9 und 18 mit einer etwa 2 mm breiten 0,1 mm dicken Barriere 15 aus Paraffinwachs versehen.

Zur Ermittlung der Plasmagewinnung werden auf die Mitte des Glasfaserpapieres 3 die in der folgenden Tabelle 3 angegebenen Blutmengen aufgetragen und nach 30 Sekunden die Benetzung der saugfähigen Schicht 9 sowie gegebenenfalls eine Übersättigung (Eindringtiefe der Erythrozyten in die Schicht 9) bestimmt. Die Ergebnisse der Tabelle zeigen, daß durch die hydrophobe Barriere die Trennung verbessert und eine vollständige Sättigung erreicht wird, so daß man bereits mit sehr kleinen Blutvolumina, beispielsweise mit Kapillarblut aus der Fingerbeere, einen solchen Test durchführen kann. Die Versuche wurden jeweils fünffach durchgeführt und die Ergebnisse gemittelt.

Tabelle 3

| Abmessungen des Glasfaservlieses (3) | getüpfeltes Blutvolumen (µl) | %uale Benetzung der Schicht 9 mit Hydrophob-Barriere | | %uale Benetzung der Schicht 9 ohne Hydrophob-Barriere | |
|---|---|---|---|---|---|
| | | Plasma $\bar{X} n = 5$ | Blut $\bar{X} n = 5$ | Plasma $\bar{X} n = 5$ | Blut $\bar{X} n = 5$ |
| 6 × 6 mm | 25 | 88 | 0 | 77 | 12 |
| | 30 | 97 | 0 | 72 | 22 |
| | 35 | 99 | 0 | 83 | 18 |
| 7 × 6 mm | 29 | 86 | 0 | 82 | 3 |
| | 35 | 100 | 0 | 87 | 21 |
| | 41 | 100 | 0 | 97 | 31 |
| 8 × 6 mm | 33 | 96 | 0 | 79 | 0 |
| | 40 | 100 | 0 | 92 | 0 |
| | 47 | 100 | 0 | 100 | 13 |

Bezugszeichenliste

1 Reaktionsschicht
2 Unterlage
3 Glasfaserpapier/Glasfaserschicht/Trennschicht
3a Glasfaserbezirk
4 Trennschicht
4a Griff
5 Netzwerk
6 durchlässige Schicht
7 durchsichtige Abdeckschicht
8 Bluttropfen/Blut
9 saugfähiges Material/Schicht
11 Löcher
12 vorgefertigte Form
13 Gefäß
14 Glasfasern
15 hydrophobe Barriere
16 Ventil
17 Kolben
18 hydrophobes Netz

**Patentansprüche**

1. Verfahren zum Abtrennen von Plasma oder Serum aus Vollblut, dadurch gekennzeichnet, daß man das Blut langsam durch eine Schicht aus Glasfasern mit einem mittleren Durchmesser von 0,2 – 2,5 µm und einer Dichte von 0,1 – 0,5 g/cm³ sickern läßt und das abgetrennte Plasma oder Serum gewinnt, wobei das Volumen des abzutrennenden Plasmas oder Serum höchstens 50%, vorzugsweise weniger als 30% vom Saugvolumen der Glasfaserschicht beträgt.

2. Verfahren nach Anspruch 1, dadurch gekennzeichnet, daß den Glasfasern synthetische organische Fasern beigemischt oder diese durch anorganische oder organische Bindemittel verfestigt bzw. miteinander verklebt sind.

3. Verfahren nach Anspruch 1, dadurch gekennzeichnet, daß die Glasfasern in einer Säule geschichtet sind und am Kopf der Säule die Aufgabe für das Blut und am Ende die Abnahme für das Plasma erfolgt.

4. Verfahren nach Anspruch 3, dadurch gekennzeichnet, daß unterhalb der Glasfaserschicht ein Ventil und darunter ein Zwischenspeicher für das Plasma vorgesehen ist, in dem sich das Plasma sammelt und aus dem es in einem zweiten Schritt durch Komprimieren ausgetrieben wird.

5. Verfahren nach Anspruch 3 oder 4, dadurch gekennzeichnet, daß man das ablaufende Plasma in einem saugfähigen Träger auffängt und trocknet und zum Nachweis der Inhaltsstoffe eluiert.

6. Verfahren nach Anspruch 3 oder 4, dadurch gekennzeichnet, daß man das ablaufende Plasma direkt in ein diagnostisches Mittel einbringt.

7. Verfahren nach Anspruch 1, dadurch gekennzeichnet, daß die Glasfaserschicht aus einem Glasfaserpapier oder Glasfaservlies besteht und Teil eines diagnostischen Mittels für den Nachweis von Inhaltsstoffen des Plasmas ist.

8. Verfahren nach Anspruch 7, dadurch gekennzeichnet, daß die Glasfaserschicht die oberste Schicht eines mehrschichtigen diagnostischen Mittels ist, welches auf einem inerten Träger befestigt ist.

9. Verfahren nach Anspruch 8, dadurch gekennzeichnet, daß die Nachweisreaktion in der untersten Schicht abläuft und durch den Träger analysiert wird.

10. Verfahren nach Anspruch 7, dadurch gekennzeichnet, daß die Glasfaserschicht und gegebenenfalls weitere Schichten über eine ablösbare Schicht mit der Reaktionsschicht verbunden sind und nach dem Durchtreten des Plasmas bzw. nach Ablauf der Reaktion mit der ablösbaren Schicht zusammen entfernt werden.

11. Verfahren nach Anspruch 10, dadurch gekennzeichnet, daß die ablösbare Schicht ein Netzwerk ist, welches neben der Reaktionsschicht mit dem Träger verbunden ist.

12. Verfahren nach Anspruch 7, dadurch gekennzeichnet, daß die Reaktionsschicht auf einem Teilbereich der Glasfaserschicht in saugfähigem Kontakt aufgeklebt oder aufgedruckt ist und das Blut auf den anderen Teilbereich aufgebracht wird.

13. Verfahren nach Anspruch 7, dadurch gekennzeichnet, daß die Reaktionsschicht über einem ersten Teilbereich der Glasfaserschicht angebracht ist, das Blut auf einem zweiten Teilbereich aufgebracht wird und die Reaktionsschicht durch Druck in Kontakt mit dem ersten Teilbereich gebracht wird, wenn dieser sich mit abgetrenntem Plasma gefüllt hat.

## Claims

1. Process for the separation of plasma or serum from whole blood, characterised in that one allows the blood to trickle slowly through a layer of glass fibres with an average diameter of 0.2 – 2.5 μm and a density of 0.1 – 0.5 g/cm³ and recovers the separated plasma or serum, whereby the volume of the plasma or serum, whereby the volume of the plasma or serum to be separated amounts to at most 50%, preferably less than 30% of the absorption volume of the glass fibre layer.

2. Process according to claim 1, characterised in that synthetic organic fibres are mixed with the glass fibres or these are consolidated or stuck to one another by inorganic or organic binding agents.

3. Process according to claim 1, characterised in that the glass fibres are stacked in a column and at the top of the column there takes place the introduction of the blood and on the end the removal of the plasma.

4. Process according to claim 3, characterised in that below the glass fibre layer there is provided a valve and thereunder an intermediate reservoir for the plasma in which the plasma collects and from which, in a second step, it is forced out by compression.

5. Process according to claim 3 or 4, characterised in that one collects and dries the plasma running off in a absorbent carrier and elutes for the detection of component materials.

6. Process according to claim 3 or 4, characterised in that one introduces the plasma running off directly into a diagnostic agent.

7. Process according to claim 1, characterised in that the glass fibre layer consists of a glass fibre paper or glass fibre fleece and is part of a diagnostic agent for the detection of component materials of the plasma.

8. Process according to claim 7, characterised in that the glass fibre layer is the uppermost layer of a multilayer diagnostic agent which is fixed to an inert carrier.

9. Process according to claim 8, characterised in that the detection reaction takes place in the lowermost layer and is analysed through the carrier.

10. Process according to claim 7, characterised in that the glass fibre layer and possibly further layers are connected via a removable layer with the reaction layer and, after the passing through of the plasma or after taking place of the reaction, are removed together with the removable layer.

11. Process according to claim 10, characterised in that the removable layer is a mesh which is connected beside the reaction layer with the carrier.

12. Process according to claim 7, characterised in that the reaction layer is stuck or pressed on to a partial zone of the glass fibre layer in absorbent contact and the blood is applied to the other partial zone.

13. Process according to claim 7, characterised in that the reaction layer is placed over a first partial zone of the glass fibre layer, the blood is applied to a second partial zone and the reaction layer is brought by pressure into contact with the first partial zone when this has filled with separated plasma.

## Revendications

1. Procédé pour l'isolement de plasma ou de sérum de sang entier, caractérisé en ce que l'on fait couler le sang lentement goutte à goutte à travers une couche de fibres de verre ayant un diamètre moyen compris entre 0,2 et 2,5 µm et une masse volumique comprise entre 0,1 et 0,5 $g/cm^3$ et en ce qu'on recueille le plasma ou le sérum isolé, le volume du plasma ou du sérum à isoler s'élevant au maximum à 50%, de préférence à moins de 30% du volume absorbant de la couche de fibres de verre.

2. Procédé selon la revendication 1, caractérisé en ce qu'on mélange les fibres de verre avec des fibres organiques synthétiques ou bien les fibres de verre sont consolidées par des liants minéraux ou organiques ou sont collées ensemble par ces liants.

3. Procédé selon la revendication 1, caractérisé en ce que les fibres de verre sont disposées en couche dans une colonne et en ce que le sang est introduit au sommet de la colonne et que le plasma est recueilli dans le bas de la colonne.

4. Procédé selon la revendication 3, caractérisé en ce qu'il est prévu une valve sous la couche de fibres de verre et sous la valve un réservoir intermédiaire pour le plasma, dans lequel le plasma se rassemble et dont il est chassé dans un second stade par compression.

5. Procédé selon la revendication 3 ou 4, caractérisé en ce que l'on recueille le plasma qui s'écoule dans un support absorbant, on le sèche et on le reprend dans un éluant pour la mise en évidence des substances qu'il contient.

6. Procédé selon la revendication 3 ou 4, caractérisé en ce que l'on introduit le plasma qui s'écoule directement dans un agent de diagnostic.

7. Procédé selon la revendication 1, caractérisé en ce que la couche de fibres de verre est un papier ou un voile de fibres de verre et fait partie d'un agent de diagnostic pour la mise en évidence de substances contenues dans le plasma.

8. Procédé selon la revendication 7, caractérisé en ce que la couche de fibres de verre forme la couche supérieure d'un agent de diagnostic à couches multiples fixé sur un support inerte.

9. Procédé selon la revendication 8, caractérisé en ce que la réaction de mise en évidence se déroule dans la couche inférieure et est analysée par le support.

10. Procédé selon la revendication 7, caractérisé en ce que la couche de fibres de verre et les couches complémentaires éventuelles sont fixées par l'intermédiaire d'une couche amovible à la couche réactionnelle et sont enlevées après le passage du plasma ou après la réaction ensemble avec la couche amovible.

11. Procédé selon la revendication 10, caractérisé en ce que la couche amovible est un réseau fixé sur le support à côté de la couche réactionnelle.

12. Procédé selon la revendication 7, caractérisé en ce que la couche réactionnelle est collée ou appuyée de façon à pouvoir exercer sa capacité absorbante, sur une partie de la couche de fibres de verre et en ce que le sang est porté sur l'autre partie.

13. Procédé selon la revendication 7, caractérisé en ce que la couche réactionnelle est placée au-dessus d'une première partie de la couche de fibres de verre, le sang est porté sur une seconde partie et la couche réactionnelle est mise en contact avec la première partie au moyen d'une pression lorsque cette dernière s'est remplie de plasma isolé.

Fig.1

Fig. 2a

Fig. 2b

Fig.3a

Fig.3b

Fig.3c

Fig.3d

Fig.3e

Fig.4

Fig.5

Fig.6

Fig.6a

Fig.6b

Fig.7

Fig.7a

Fig.8a

Fig.8b

Fig.9

8

13

14

8

14

13

16

14

Fig.10

17

8

13

14

Fig.11

Fig.12

Fig.13

Fig.14

Fig. *15 a*

Fig. *15 b*

Fig.16 a

Fig.16 b

Fig.17a

Fig.17b

Fig. *18*

Fig. *19*